(19) **Europäisches Patentamt**
**European Patent Office**
**Office européen des brevets**

(11) **EP 2 812 309 B1**

(12) **EUROPEAN PATENT SPECIFICATION**

(45) Date of publication and mention
of the grant of the patent:
**26.05.2021 Bulletin 2021/21**

(21) Application number: **13746269.3**

(22) Date of filing: **06.02.2013**

(51) Int Cl.:
*C07C 227/40* (2006.01)      *B01D 15/08* (2006.01)
*C07C 7/12* (2006.01)      *B01D 15/18* (2006.01)
*C12P 13/08* (2006.01)

(86) International application number:
**PCT/KR2013/000945**

(87) International publication number:
**WO 2013/119034 (15.08.2013 Gazette 2013/33)**

(54) **METHOD FOR CONTINUOUS SEPARATION OF VALINE**

VERFAHREN ZUR KONTINUIERLICHEN ABSCHEIDUNG VON VALIN

PROCÉDÉ DE SÉPARATION CONTINUE DE VALINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.02.2012 US 201261595527 P
07.11.2012 KR 20120125739**

(43) Date of publication of application:
**17.12.2014 Bulletin 2014/51**

(73) Proprietors:
• **CJ Cheiljedang Corporation
Seoul 100-400 (KR)**
• **Industry-University Cooperation Foundation
Hanyang University
Seoul 133-791 (KR)**

(72) Inventors:
• **LEE, Chong Ho
Seoul 157-794 (KR)**
• **KANG, Seung Hoon
Bucheon-si
Gyeonggi-do 421-220 (KR)**
• **YU, Jae Hun
Seoul 157-210 (KR)**

• **KIM, Yu Shin
Seoul 157-200 (KR)**
• **MUN, Sung Yong
Seoul 140-728 (KR)**
• **NAM, Hee Geun
Seoul 157-910 (KR)**
• **PARK, Chan Hun
Seoul 135-280 (KR)**

(74) Representative: **Fuchs Patentanwälte
Partnerschaft mbB
Westhafenplatz 1
60327 Frankfurt am Main (DE)**

(56) References cited:
**EP-A1- 1 106 602      CN-A- 101 948 399
KR-A- 20070 067 926      KR-A- 20120 009 962
US-A1- 2012 071 691**

• **SUNGYONG MUN: 'Strategy of rearranging the
port locations in a three-zone simulated moving
bed chromatography for binary separation with
linear isotherms' JOURNAL OF
CHROMATOGRAPHY A vol. 1230, 04 February
2012, pages 100 - 109, XP028463817**

EP 2 812 309 B1

## Description

**Technical Field**

The present invention relates to a method for continuous separation of valine, and more specifically to a method for continuous separation of valine from a mixture comprising amino acids such as leucine, isoleucine, etc.

**Background Art**

Valine is an L-amino acid having a formula of $HO_2CCH(NH_2)CH(CH_3)_2$ and used as one of the main raw materials in medicine and in cosmetics. It is also one of the important ingredients utilized for animal feed. For this reason, there is a daily increase of interest within commercial markets focusing on the applications of valine.

The production of valine is generally achieved by a procedure of fermentation of *Corynebacterium*. In this regard, a matter of note is that impurities are also obtained along with valine during the fermentation procedure. These impurities include salt, alanine, leucine, isoleucine, etc., and all of these impurities are required to be separated from valine.

Separation methods for valine used in the prior art include an ion exclusion-chromatography method (Japanese Laid-open Patent Publication No. 1987-255453), a crystallization method using a precipitant (Japanese Laid-open Patent Publication No. 1996-333312, Japanese Laid-open Patent Publication No. 1998-237030, and U.S. Patent No. 6,072,083), a chemical reaction method (U.S. Patent No. 4,263,450), and the like.

Further, CN 101 948 399 A discloses a method for continuously separating and purifying valine in fermentation liquor by using simulated moving bed chromatography. In the method, a simulated moving bed chromatography separating device suitable for the composition characteristic of valine fermentation liquor is designed. Water is used as the mobile phase and a resin special for valine separation is used as the stationary phase. The valine fermentation liquor contains as impurities alanine and leucine.

However, among the separation methods, the ion exclusion-chromatography method has disadvantages in that the separation of amino acids such as leucine and isoleucine is difficult, a lot of waste water is generated, and a post-treatment process such as crystallization, etc. is additionally needed. Also, since the crystallization method using the precipitant needs a process for removing the precipitant, it has the disadvantage that the process becomes complex and that a post-treatment process for purification is additionally needed. Further, since the chemical reaction method needs a concentration and hydrolysis process, it has the disadvantage that the process is complex and utilizes lot of solvents, and thus a number of labs are needed in the post-treatment process for recovering it, and the costs thereof in the purification process are accordingly increased.

Meanwhile, the chromatographic separation process is a separation process based on an adsorbent and is widely used in processes for the separation and purification of a plurality of bioproducts, and according to the application is broadly divided into a batch chromatography process and a continuous Simulated Moving Bed (SMB) chromatography process. The SMB process was initially developed for the separation of petrochemicals by UOP, USA in 1961, and it has been reported that its performance and separation efficiency is far superior to that of the batch process. Due to the superiority of the SMB process, it has been expanded to the separation and purification of high-value added products such as sugar materials, chiral compounds, bioproducts, medicines, etc. as well as the separation and purification of petrochemicals.

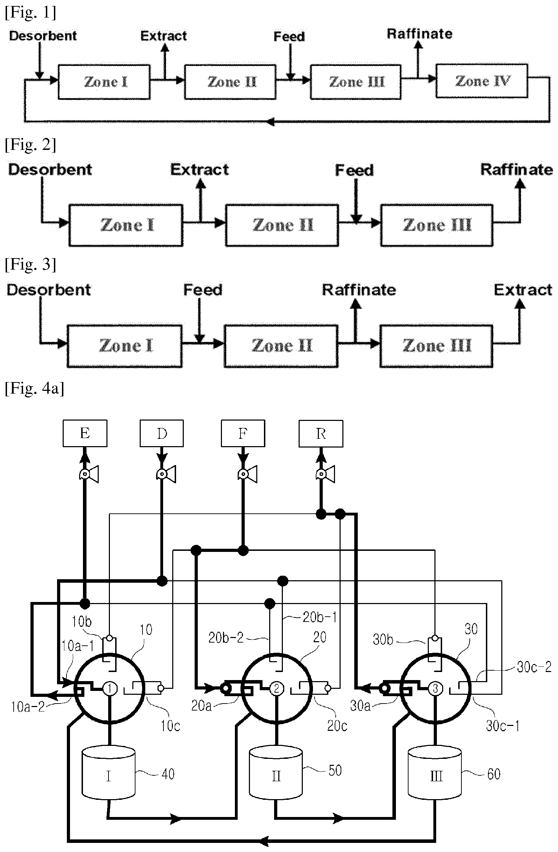

Most conventional SMB processes (four-zone SMB) are composed of a structure having four zones (columns) and four ports, as shown in Fig. 1, and each of the mixtures to be separated and the solvent (Desorbent) flow into a port (Feed) for the mixture to be separated and a desorption port (Desorbent) among the four ports. In addition, materials having weak adsorptive power and strong adsorptive power are separated and then obtained via Raffinate port (Raffinate) and Extract port (Extract). In order for the processes corresponding to the input of the mixture and the solvent to be separated and the recovery of the two components above to be continuously performed, the four ports are moved along the direction in which the solvent flows at a constant rate of switching.

However, the four-zone SMB process needs at least four columns. Generally, since both the valves of each column and the adsorbent are expensive, it is preferable to minimize the number of the columns if possible.

For this reason, a SMB process having three chromatography zones (three-zone SMB) as shown in Fig. 2 has been introduced. Since it uses three zones, the minimum number of columns can be reduced from four to three. In addition, the positions of the four ports are disposed in a manner identical in terms of structure relative to that of the conventional SMB structure having four chromatography zones.

However, the low-affinity component having weak adsorptive power and thus moving fast, such as valine in the above three-zone SMB process, is recovered via the Raffinate port as shown in Fig. 2 but, since there is no enrichment zone for the raffinate as in Fig. 2, excessive dilution is unavoidable. That is, since the concentration of valine recovered is lower than the valine concentration in the mixture to be separated, a side effect occurs in that operation cost for the post-treatment process following the SMB separation process is increased. Therefore, the application of the three-zone SMB process found in the new system is needed, in that it is able to overcome the side effect.

Accordingly, during research in consideration of the above-mentioned matters, the present inventors have ascertained that valine can be continuously and efficiently separated from a mixture comprising amino acids such as leucine, isoleucine, etc., through an apparatus using a simulated moving bed chromatography process, thereby completing the present invention.

**Disclosure of Invention**

**Technical Problem**

An object of the present invention is to provide a method for continuous separation of valine from the mixture comprising amino acids such as leucine, isoleucine, etc.

**Solution to Problem**

In order to resolve the above problem, the apparatus used for the separation of valine in the present invention includes a Desorbent port (D), a Feed port (F), a Raffinate port (R), an Extract port (E), three rotary valves (10, 20, 30), and three chromatography zones (40, 50, 60) connected to each of three rotary valves (10, 20, 30), as shown in Figs. 4a - 4c.

Three rotary valves (10, 20, 30) are equipped with three connection ports (10a, 10b, 10c)(20a, 20b, 20c)(30a, 30b, 30c), respectively, and only any one connection port of each rotary valve (10, 20, 30) is opened along with the rotation of the rotary valves (10, 20, 30), and is in fluid communication with the Desorbent port (D), the Feed port (F), the Raffinate port (R) and the Extract port (E).

That is, the flow passage connected to the Desorbent port (D), the Feed port (F), the Raffinate port (R) and the Extract port (E) have three branches, respectively, and thus, these are all connected to three rotary valves (10, 20, 30), and afterward are connected to a certain rotary valve concurrent with the opening of any one of the connection ports.

Hereinafter, the specific mode of operation is explained.

Fig. 3 is a schematic diagram for a constitution of the simulated moving bed process (three-zone SMB) having three chromatography zones used in the present invention. As can be seen from Fig. 3, since the displacement order of the ports is the Desorbent port, the Feed port, the Raffinate port and the Extract port, respectively, it is possible to operate the enrichment zone in such a way as to prevent the dilution of the Raffinate concentration. As a result, it has an advantage in that the degradation of the Raffinate concentration, which is a problem of the conventional three-zone SMB, can be prevented. In addition, since it has the structure wherein the Desorbent port and the Feed port are connected through one column, there is an advantage in that the amount of the solvent used can be reduced.

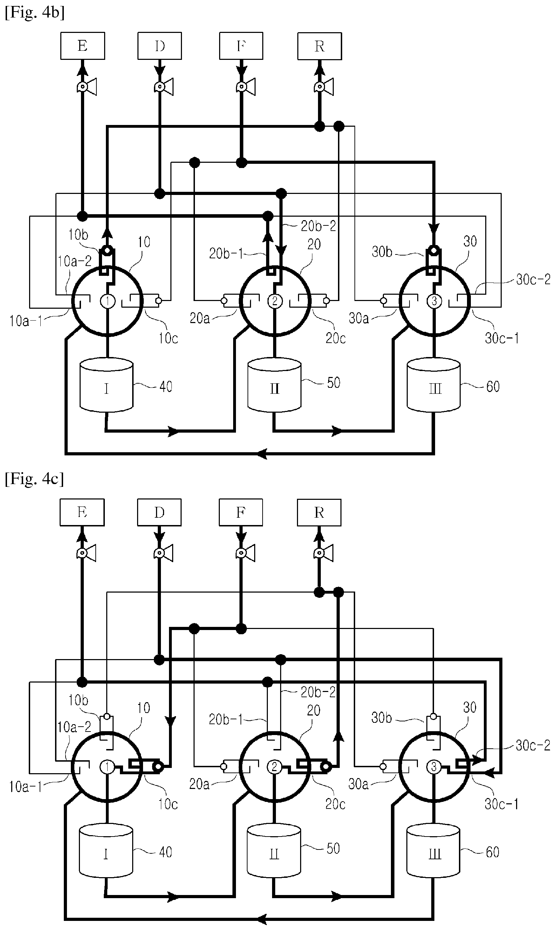

Further, the present invention uses an apparatus in which the process as disclosed in Fig. 4 is continuously in operation. Fig. 4a depicts the apparatus in the first position, Fig. 4b depicts the apparatus in the second position, and Fig. 4c depicts the apparatus in the third position. The first, second and third positions are rotated between continuously. That is, the apparatus used for the present invention has a mode of operation wherein the chronological consists of the first position, the second position, the third position, and then a return to the first position again.

A setting to a certain position is made by the rotation of rotary valves (10, 20, 30). That is, the first connection ports (10a, 20a, 30a) of rotary valves (10, 20, 30) are opened and are thus set to the first position, the second connection ports (10b, 20, 30b) are opened by rotating the rotary valves (10, 20, 30) and thus are set to the second position, and the third connection ports (10c, 20c, 30c) are opened by rotating the rotary valves (10, 20, 30) again and are set to the third position. If the rotary valves (10, 20, 30) are rotated again, they are set to the first position again.

Meanwhile, hereinafter, some connection ports (10a, 20b, 30c) are separately shown with regard to inlet ports (10a-1, 20b-1, 30c-1) and outlet ports (10a-2, 20b-2, 30c-2) and explained for purposes of comprehension.

In the first position, as shown in Fig. 4a, only the first connection ports (10a, 20a, 30a) of the rotary valves (10, 20, 30) are opened, and the second connection ports (10b, 20b, 30b) and the third connection ports (10c, 20c, 30c) are closed.

In the first position, the desorbent port (D) is connected to the first rotary valve (10), the Feed port (F) is connected to the second rotary valve (20), the Raffinate port (R) is connected to the third rotary valve (30), and the Extract port (E) is connected to the first rotary valve (10).

Accordingly, the desorbent flowed from the Desorbent port (D) passes through the first rotary valve (10) and the first chromatography zone (40) and then flows in to the second rotary valve (20).

The mixture to be separated that is flowed from the Feed port (F) is flowed to the second rotary valve (20) together with the desorbent passed through the first chromatography zone (40), and then is passed through the second chromatography zone (50).

The mixture to be separated in the present invention comprises valine, and additionally comprises salt, alanine, leucine, isoleucine, etc. as impurities therein. The separation of the constituents of the mixture to be separated is achieved by a difference in flow rates after passing through the second chromatography zone (50). Valine is a low-affinity component that has a weaker adsorptive power than that of other impurities, and thereby moves faster. Therefore, the mixture to be separated is then separated into valine and other materials, and each of them flows into the third rotary valve (30) with the time difference.

Valine intended to be separated flows out via the Raffinate port (R) but, other materials are flowed out to the Extract port (E) by passing through the third chromatography zone (60) and then the first rotary valve (10).

After the set time (i.e., the rotational time interval of the rotary valve) has elapsed, the rotary valves (10, 20, 30) are rotated and then changed to the second position as shown in Fig 4b. A criterion of the set time is described below.

In the second position, shown in Fig. 4b, only the second connection ports (10b, 20b, 30b) of rotary valves (10, 20, 30) are opened, and the first connection ports (10a, 20a, 30a) and the third connection ports (10c, 20c, 30c) are closed.

Upon comparison with the first position, the rotary valves (10, 20, 30) connected to ports (D, F, R, E) are moved sequentially one by one in the second position.

That is, in the second position, the desorbent port (D) is connected to the second rotary valve (20), the Feed port (F) is connected to the third rotary valve (30), the Raffinate port (R) is connected to the first rotary valve (40), and the Extract port (E) is connected to the second rotary valve (20).

Accordingly, the desorbent flowed in from the desorbent port (D) is flowed in to the third rotary valve (30) after passing through the second rotary valve (20) and the second chromatography zone (50).

The mixture to be separated that is flowed in from the Feed port (F) is flowed in to the third rotary valve (30), together with the desorbent passed through the second chromatography zone (50), and is passed through the third chromatography zone (60).

However, in this case, the remaining materials of the mixture to be separated that have flowed in from the first position as shown in Fig. 4a have not yet been flowed into the third rotary valve (30), due to the difference in flow rates. In order to accomplish this, the rotation time interval mentioned above is controlled.

Therefore, the mixture to be separated that is flowed into the third rotary valve (30) at the second position passes through the third chromatography zone (60) and then the separation of the mixture to be separated is made due to the difference of the flow rates and is then separated into valine and other materials, wherein the valine intended to be separated is flowed out via the first rotary valve (10) and the Raffinate port (R) while remaining materials are combined with other materials that were separated in the first position but were not yet flowed into the third rotary valve (30), to then pass through the second rotary valve (20) more effectively and are then flowed into the Extract port (E).

After a lapse of the predetermined time period, the rotary valves (10, 20, 30) are rotated and changed to the third position, as shown in Fig. 4c.

In the third position, shown in Fig. 4c, only the third connection ports (10c, 20c, 30c) of the rotary valves (10, 20, 30) are opened, and the first connection ports (10a, 20a, 30a) and the second connection ports (10b, 20b, 30b) are closed.

Upon comparison with the second position, the rotary valves (10, 20, 30) connected to ports (D, F, R, E) are sequentially moved one by one in the third position.

Identical to the explanation of the first and second positions, the mixture to be separated are flowed in from the Feed port (F) and then are flowed into the first rotary valve (10) together with the desorbent passed through the third chromatography zone (60), and then passed through the first chromatography zone (40), wherein, since in this case the remaining materials of the mixture to be separated are flowed in at the second position as shown in Fig 4b and are not yet flowed into the second rotary valve (20) due to the difference of flow rates, they are also combined together and flowed into the Extract port (E) after passing through the third rotary valve (30).

Valine intended to be separated in the mixture to be separated is separated to the Raffinate port (R) after passing through the second rotary valve (20).

After the predetermined time period, the rotary valves (10, 20, 30) are rotated and changed to the first position as shown in Fig. 4a, and the procedure is continuously repeated.

As the adsorbent used in the first, second and third chromatography zones in the present invention, a porous polymer resin is used, consisting of an insoluble polystyrene divinylbenzene polymer material. Since the above resin has a broad surface area, a unique pore size and volume distribution, it can be preferably applied for the purification of various materials, and in particular pharmaceutical compounds. Amberchrom CG161 (Rohm & haas) or Chromalite PCG Series (Purolite), etc. can be used as a specific example. The above commercially available resin has a diameter of a particle of 10 - 300 $\mu$m, a surface area of 700 - 900 m$^2$/g, a pore size of 100 - 200 Å, a pore size and volume distribution of 0.7 - 1.5 ml/g, and a uniformity coefficient of less than 2.

The mixture to be separated is a mixture comprising valine, isoleucine or leucine being included in the branched amino acids together with valine, and preferably may be valine fermentation liquor obtained through the fermentation of a microorganism. The specific examples of the present invention utilize the mixture in which the purified valine, isoleucine and leucine are artificially mixed, and the valine fermentation liquor obtained by the microorganism fermentation. This is because there is an infrequent case in which only valine is produced in the fermentation of the microorganism. In most cases, isoleucine and leucine are additionally produced together.

By the continuous separation apparatus used for the present invention, since valine intended to be separated in the mixture to be separated is flowed out via the Raffinate port (R), and the remaining materials are more effectively separated by the rotation of rotary valves (10, 20, 30) as well as the difference of the flow rate and are flowed out via the Extract port (E), thereby having valine be continuously separated.

Additionally, through control of the continuous separation apparatus of valine of the present invention, it can be controlled by regulating the flow rates of the desorbent port (D), the Feed port (F) and the Raffinate port (R), and also by regulating the rotation velocity (switching time) of the rotary valve. The flow rate of each port can be regulated in consideration of the intrinsic parameters of the column (adsorption coefficient, mass transfer coefficient, porosity, material environment, etc).

In addition, the present invention provides the continuous separation method of valine characterized in that it comprises the steps of flowing the desorbent using the desorbent port, flowing the mixture comprising valine into the Feed port, and recovering valine from the Raffinate port, by way of the method for the continuous separation of valine by using the continuous separation apparatus of the valine. The desorbent is preferably water. In addition, it is characterized in that the purity of valine recovered by the method is 90% to 99%.

According to one example of the present invention, as a result of the separation of the mixture to be separated comprising valine, leucine and isoleucine, by using the continuous separation apparatus of valine according to the present invention, the yield of the recovered valine was about 98% or more, the purity was about 98% or more, and thus, it was verified that valine could be effectively separated from the mixture.

**Advantageous Effects of Invention**

The present invention can continuously separate valine from the mixture comprising amino acids such as leucine, isoleucine, etc. while having high purity and yield, by way of the apparatus using the simulated moving bed chromatography process.

**Brief Description of Drawings**

Fig. 1 depicts the schematic diagram of the Simulated Moving Bed (four-zone SMB) chromatographic process having the conventional four chromatography zones.

Fig. 2 is the schematic diagram of the Simulated Moving Bed (three-zone SMB) chromatographic process having the conventional three chromatography zones.

Fig. 3 is the schematic diagram of the Simulated Moving Bed chromatographic process having three chromatography zones (three-zone SMB) used in the present invention and the process is developed based on the port disposition mode which is differentiated from the conventional three-zone SMB.

Fig. 4 shows a mimetic diagram of the apparatus for the three-zone SMB process used in the present invention. Figs. 4a, 4b and 4c show the connections of each zone according to the rotation of rotary valves, respectively.

Fig. 5 shows the pulse test results for selecting the adsorbent. Wherein, (a) represents the result for Amberchrom CG161C, (b) represents the result for Amberlite CG71C, (c) represents the result for DIAION SK1B, and (d) represents the result for Amberlite XAD-7HP.

Fig. 6 shows the result of a stepwise frontal test for Amberchrom CG161C adsorbent. Wherein, (a) represents the result for isoleucine, (b) represents the result for leucine, and (c) represents the result for valine.

Fig. 7 shows the adsorptive equilibrium data (q vs. C) on Amberchrom CG161C adsorbent. Wherein, (a) represents the data for valine, (b) represents the data for leucine, and (c) represents the data for isoleucine.

Fig. 8 shows a comparison between the frontal test result of the mixture and the simulation result. Wherein, (a) represents the comparison of the result for leucine, (b) represents the comparison of the result for isoleucine, and (c) represents the comparison of the result for valine.

Fig. 9 shows the result of HPLC concentration analysis for outflow solution discharged through the SMB desorbent port, as SMB test result. Wherein, (a) represents the result for Raffinate concentration analysis, and (b) represents the result for the extract concentration analysis.

Fig. 10 shows a HPLC raw data chromatogram for the sample obtained from SMB test. Wherein, (a) represents the chromatogram of the mixture to be separated, (b) represents that of raffinate, and (c) represents that of the extract.

Fig. 11 shows a column profile (60.5 step), as a SMB test result. Wherein the black line represents the simulated result of valine, the gray line represents the simulated result of isoleucine, the black dotted line represents the simulated result of leucine, the circle represents the test result of valine, the quadrangle represents the test result of isoleucine, and the diamond represents the test result of leucine.

Fig. 12 shows the HPLC concentration analysis result of outflow solutions discharging via the Raffinate port, as the SMB test result conducted based on Chromalite PCG-600 adsorbent.

Fig. 13 shows the HPLC concentration analysis result for outflow solutions discharged via the Extract port, as the SMB test result conducted based on Chromalite PCG-600 adsorbent.

Fig. 14 shows a column profile (44.5 step), as SMB test result conducted based on Chromalite PCG-600 adsorbent. Wherein, the black line represents the simulated result of valine, the gray line represents the simulated result of leucine, the circle represents the test result of valine, and the triangle represents the test result of leucine.

## Mode for the Invention

Hereinafter, it is intended to explain the constitution and effects of the present invention in detail via Examples, but these Examples are intended only to illustrate the description of the present invention.

## Approach: Investigation for the chromatographic application of Valine purification

1) Model-based design approach

There are two matters that are initially considered upon developing a continuous process for a new separation system. The first is the minimization of the cost and the period required for development. The second is the conditions providing the best output productivity and the best separation efficiency, by maintaining the optimum state of the process to be developed. In order to satisfy both conditions as above, one should precisely grasp the adsorption and material-transferring phenomenon based on the detailed model, and also obtain various parameters related thereto. The method of process design based on this detailed model and parameters is referred to as a "model-based design approach". Also, the present invention has developed a SMB process in carrying out the continuous separation of valine according to the approach.

2) Computer Simulation

One of the key stages of model-based design approach is a computer simulation. This refers to the procedure of obtaining the solution by solving the detailed model equation for the adsorption and material-transferring phenomenon of each component in a column by way of a numerical analytic method. This numerical analytic method is carried out by using a computer, since it requires a vast number of calculations.

There are many kinds of column model equations to be used in a simulation, and among them the lumped mass-transfer model (Kang, S.H. et al., Process Biochem., 2010, 45, 1468-1476) is determined as the simulation model of the present invention in consideration of accuracy and efficiency. The computer simulation based on lumped mass-transfer model was utilized in the assessment of separation efficiency of the SMB process as well as the measurement and assessment of base parameter of each amino acid component. Furthermore, this model equation is utilized in the manufacture of the SMB optimization computer tool.

3) SMB Optimization Tool

Another key role in the model-based design approach that is utilized after the computer simulation is played by the SMB optimized computer tool. This tool is used in obtaining the optimal operating conditions of SMB process to be developed. The matter first necessary for manufacturing this optimized tool is an optimization algorithm. Conventionally, it is known that a gene algorithm based on stochastic theory is the most efficient in the complex form of the process optimization such as SMB (Lee, K.B. et al., AIChE J., 2008, 54, 2852-2871).

Also, the present invention has established a SMB optimized computer program based on a gene algorithm for optimization of continuous separation of valine. The gene algorithm itself has been developed several times in the interim, and the NSGA-II-JG algorithm (Lee, K.B. et al., AIChE J., 2008, 54, 2852-2871), which can be said to be the latest gene algorithm in the manufacturing stage of the optimization tool of the present invention, is adopted as the basic algorithm.

The method for preparing a SMB optimized tool codes the optimized algorithm by using visual basic application (VBA) language installed in Microsoft Excel software, and allows for the calculations of the detailed model equation and the NSGA-II-JG algorithm to be simultaneously carried out therein.

**Preparation of Experiment**

1) Materials

Valine and leucine, among three amino acid components constituting the mixture to be separated were purchased from Fluka, and isoleucine was purchased from Sigma. Water used to dissolve amino acid was tertiary Distilled Deionized Water (DDW) and obtained through the Milli-Q system (Millipore). Adsorbent used in the experiment was Amberchrom CG161C (Rohm & haas), Amberlite CG71C (Sigma Aldrich), DIAION SK1B (Mitsubishi Chemical), Amberlite XAD-7HP (Sigma Aldrich). Methanol used in HPLC concentration analysis was purchased from Burdick & Jackson Co. (Muskegon, MI).

An Omnifit glass column used in the adsorbent selection experiment and the measurement experiment of the basic parameter of each material was purchased from Biochemical Fluidics Co. (Boonton, NJ), and in the former experiment the column having 11.6 cm of length and 1.5 cm of diameter was used, and in the latter experiment the column having 21.7 cm of length and 2.5 cm of diameter was used.

2) Equipment

- Single column experimental equipment

Young-Lin SP930D pump and Young-Lin UV730D detector were used in the adsorbent selection experiment. The control and data processing of these two pieces of equipment was made by Autochro-3000 software. An injector used in injecting the amino acid pulse into the column each filled with adsorbent was the Rheodyne 7725i injector, and the injection volume was 100 $\mu\ell$.

In the measurement experiment of the basic parameter of each amino acid conducted after completing the adsorbent selection, FPLC P-920 pump, Waters 486 UV detector, and Amersham FPLC collector (Frac-900) were used. The control and data collection of each of the detailed devices were made through Unicorn 5.1 software.

- Three-zone SMB apparatus for continuous separation of valine

The apparatus as shown in Fig. 4 was self-assembled and used. The flow rate of the desorbent of the completed SMB experimental apparatus and the flow rate of the mixture to be separated were controlled by using the Young-Lin SP930D pump and the raffinate flow rate were controlled by the Ismatec MCP-CPF ISM 919 pump. In order to generate the effect wherein four ports are periodically moved, a ST Valco rotary valve (VICI, Houston, TX) was used. The mimetic diagrams of the ST valve are represented in Figs. 4a-4c. A Valco rotary valve used in the experiment was automatically controlled through Labview 8.0 software.

- HPLC concentration analysis device (analyzer)

In order to measure the concentration of the material collected through SMB experiment, a HPLC concentration analyzer was used. As the column for the analysis, Waters Symmetry C-18 column (250 × 4.6 mm ID, particle size 5 $\mu$m) was used. The collection of all data related to HPLC concentration analysis was treated by using Waters Millennium software. The flow rate of the mobile phase was controlled by using the Waters 515 HPLC pump. The sample for the concentration analysis was injected into the column for analysis by using a Rheodyne 9725i injector, and the injection volume was 20 $\mu\ell$. The detection of a sample was conducted by using a Waters 996 PDA detector.

In the concentration analysis of the sample obtained from a frontal test of valine, a Young-Lin SP930D pump and a Young-Lin UV730D detector were used. The control and data processing of the Young-Lin pump and detector were conducted by Autochro-3000 software. The sample obtained from the experiment was injected into the column for the analysis by using a Rheodyne 7725i injector, and the injection volume was 20 $\mu\ell$.

**Example 1: Selection of an adsorbent suitable for separation of valine**

1) Experimental Method

The experiment to select the most suitable adsorbent was conducted by using four kinds of conventional adsorbents that have the possibility of separating leucine and isoleucine from valine, such as Amberchrom CG161C (Rohm & haas), Amberlite CG71C (Sigma Aldrich), DIAION SK1B (Mitsubishi Chemical), Amberlite XAD-7HP (Sigma Aldrich), through the manner of a series of pulse tests.

The conditions for the pulse test experiments were as follows: Each of the concentrations of three amino acids was 2 g/L, and the injection volume was 100 $\mu\ell$. The flow rate of the mobile phase was 2 ml/min.

2) Experimental Results

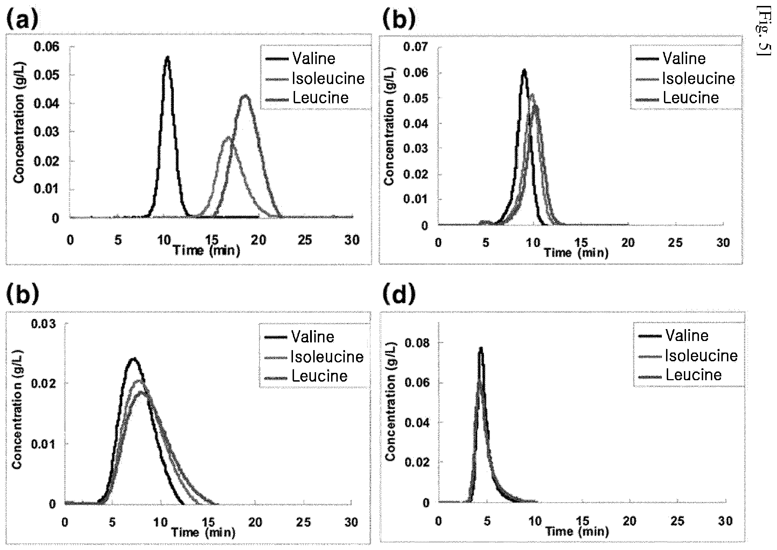

The results are represented in Fig. 5. As represented in Fig. 5, it could be ascertained that in case of other adsorbents other than the Amberchrom CG161C adsorbent, the selective separation of valine is difficult. It is ascertained from the above results that the Amberchrom CG161C adsorbent is most suitable for establishing SMB process for separating valine.

**Example 2: Measurement of porosity of Amberchrom CG161C column**

1) Experimental method

The porosity of the Amberchrom-CG 161 C adsorbent as selected in Example 1 was measured. At first, the experiment that fills the column with the adsorbent, and then measures an inter-particle porosity ($e_b$) was conducted. Although it is conducted in the same manner as Example 1, in this instance a tracer molecule suitable for measuring the inter-particle porosity was injected into the column instead of the material to be separated, unlike as in Example 1. The tracer molecule used was blue dextran, and the injection concentration was 1 g/L, the flow rate was 4 ml/min and the injection volume was 500 $\mu\ell$.

2) Experimental results

From the experimental results, the value of the inter-particle porosity of the column was 0.391. The intra-particle porosity, which is one of the important information used the value which is reported in the reference, was 0.737 (Nam, H.G. et al., Process Biochem., 2011, 46, 2044-2053).

**Example 3: Measurement of the basic parameter of valine, leucine, isoleucine on Amberchrom CG161C**

- **Measurement of adsorption coefficient**

1) Experimental method

A multiple frontal test was performed in order to measure the basic parameters of valine, leucine, isoleucine that correspond to each component of the mixture which is a subject of the separation. The multiple frontal test is the experiment that is carried out to obtain the adsorption equilibrium data for each material to be separated, and to define the adsorption model equation and determine the relevant adsorption parameters based on the obtained data.

Specifically, a multiple frontal test used two pumps, and one of the pumps was filled with DDW, and another pump was filled with valine, leucine or isoleucine solution. Then, valine, leucine or isoleucine solution was continuously injected into the column until the equilibrium between the adsorbent phase and mobile phase in the column was achieved. In the equilibrium state, since all of the concentrations between the adsorbent particle and the particle as well as the concentration inside the adsorbent are maintained similar to the concentration of valine, leucine, or isoleucine solution injected, the adsorbent concentration on the adsorbent can be immediately investigated by establishing a simple Mass Balance Equation. After the concentration inside the column reaches an equilibrium state, a new equilibrium state is allowed to be maintained by increasing the ratio of the mixture solution which is the subject of the separation, compared to that of the previous stage. Again, the adsorption concentration on the adsorbent is calculated by establishing a Mass Balance Equation for this equilibrium state. While progressing the multiple frontal test, a step time for each amino acid component is set as 2-3 times the residence time obtained from the results of the pulse test.

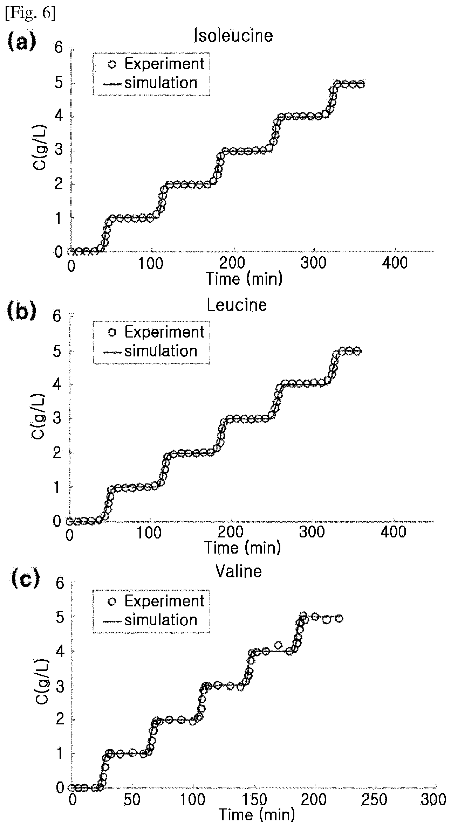

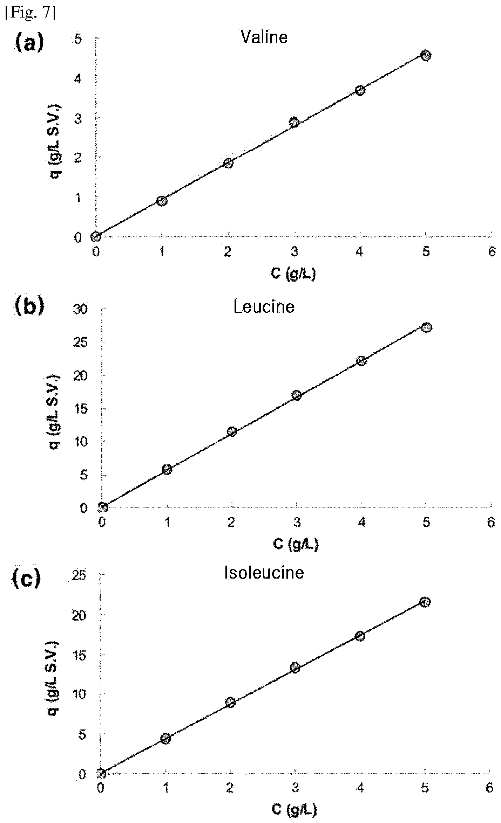

Also, the multiple frontal test for each amino acid was conducted as a total of five steps, and the step time is set as 40 min for valine, 70 min for leucine, and 70 min for isoleucine. In addition, the concentration of all samples was 5 g/L, and the flow rate was constantly maintained as 4 ml/min. The experiments for leucine and isoleucine were conducted in waves of 205 nm, and 218 nm at UV detector. On the other hand, in the case of valine, a direct HPLC analysis manner (the manner sampling the column effluents one by one and then analyzing the concentrations of them by using HPLC device), which is not an on-line monitoring manner as used in the leucine and isoleucine experiments was adopted. The HPLC analyzing conditions are that 10% aqueous methanol solution was used as the mobile phase, the injection volume was 20 $\mu\ell$, and the flow rate was 0.5 ml/min. Before the analysis, a self calibration curve was secured by using a standard solution. The results are represented in Fig. 6, the adsorption equilibrium data is calculated based on these results, and the calculated results are represented on Fig. 7.

2) Experimental Results

As shown in Figure 7, all of valine, leucine and isoleucine represent the linear adsorption relationship on Amberchrom CG161C. Therefore, each of the amino acid components is modeled by the linear adsorption equation, and the linear adsorption coefficient was determined therefrom. The values of the determined adsorption coefficients are represented in Table 1, as below.

- **Determination of a mass-transfer coefficient**

1) Experimental method

A mass transfer coefficient which can be referred to as another important basic parameter together with the adsorption coefficient was determined as per the following method.

At first, an axial dispersion coefficient and film mass-transfer coefficient were respectively expected by using Chung & Wen correlation (Chung, S. F. et al., AIChE J., 1968, 14, 857-866) and Wilson & Geankopolis correlation (Wilson, E. J. et al., Ind. Eng. Chem. Fundam., 1966, 5, 9-14), and a molecular diffusivity was calculated by using Wilke & Chang correlation (Wilke, C.R. et al., AIChE J.. 1955, 1, 264-270). Also, intra-particle diffusivity was determined while fitting the frontal experimental data and the simulation results based on the lumped mass-transfer model each other.

2) Experimental results

The values of the molecular diffusivity and the intra-particle diffusivity determined above are represented in Table 1 as below:

Table 1

[Table 1]

| | Linear adsorption coefficient | Intra-particle diffusivity ($cm^2$/min) | Molecular diffusivity ($cm^2$/min) |
|---|---|---|---|
| leucine | 5.527 | $5.40 \times 10^{-5}$ | $5.40 \times 10^{-4}$ |
| isoleucine | 4.361 | $3.00 \times 10^{-4}$ | $4.35 \times 10^{-4}$ |
| valine | 0.926 | $3.00 \times 10^{-4}$ | $5.10 \times 10^{-4}$ |

In order to ascertain the suitability of the values of the adsorption and mass transfer coefficient as determined above, the results of model simulation and frontal experimental results substituting the values were compared as shown in Figure 6.

As shown in Figure 6, it can be ascertained that the simulation results and the experimental data coincide well. Therefore, the value of the basic parameter as determined above can be sufficiently utilized for the optimization of SMB process, as conducted later.

**Example 4: Investigation of the basic parameter by a frontal experiment of the mixture**

1) Experimental method

A frontal experiment of the mixture was carried out in order to investigate the basic parameter value determined in Example 3. In this experiment, the frontal experiment was conducted by using the mixture solution comprising all of three amino acids as the mixture to be separated, unlike as in Example 3.

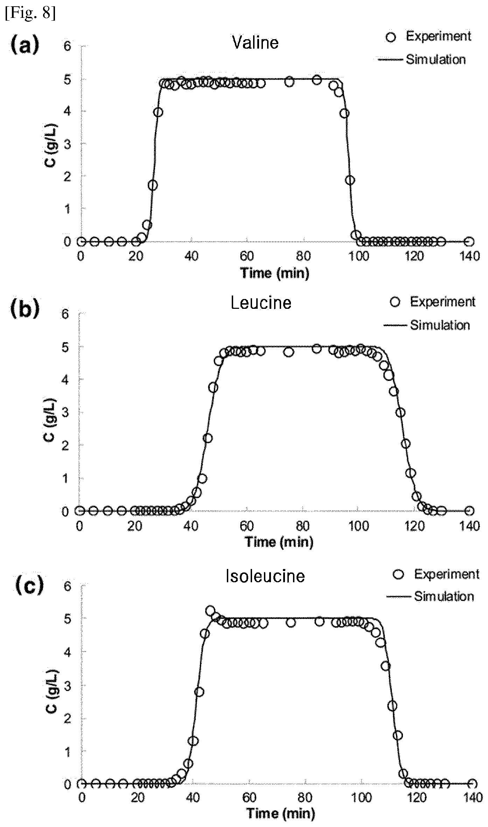

Also, the model simulation was conducted on grounds similar to those of the basic parameter values determined in Example 3 and the mixture frontal experimental conditions. Additionally, the obtained simulation results were compared to the frontal experimental data of the mixture, and the results are represented in Figure 8.

2) Experimental results

As shown in Figure 8, it can be ascertained that the experimental data coincided well with the simulation results. This means that the basic parameter values determined in Example 3 can well explain the behavior of each amino acid in the column in the state of being in the mixture as well as in the state of being a separate component. Furthermore, it means that interaction between each of the amino acids is rarely present.

**Example 5: Optimization of the process for continuous separation of valine**

The matter focused upon in the optimization procedure is to maximize the productivity of valine while assuring the high purity and high yield of valine that is the target of the separation. Wherein the productivity of valine is defined as per the following equation:

$$- \text{Productivity of valine} = Q_{raf} * C_{raf,valine}$$

(wherein, $Q_{raf}$ is the flow rate in the Raffinate port and, $C_{ref,valine}$ means the concentration of valine in the Raffinate port.)

The productivity of valine was established as an objective function and the purity and yield of valine were established as a constraint, to conduct the optimization procedures. The concrete optimization procedures are summarized as follows:

$$\text{Max J} = \text{Productivity} [Q_{feed}, Q_{raf}, t_{sw}]$$

Subject to Valine purity = 98%
Valine yield = 98%
Fixed variables $Q_{des}$ = 5 mL/min
$C_{feed}$ for each component = 5 g/L
$L_C$ = 21.7 cm, $d_C$ = 2.5 cm
(wherein, $Q_{feed}$ and $Q_{des}$ independently refer to the flow rate of the mixture to be separated and the flow rate of the desorbent, respectively, and $t_{sw}$ refers to the switching time.)

In order to optimize the separation process of valine using the three-zone SMB device of the present invention according to the equation, the optimized computer program based on the NSGA-II-JG algorism was self-coded, and the computer program was connected to an Aspen simulator to optimize the relevant process. The results are represented in Table 2 as below.

Table 2

[Table 2]

| Inlet and outlet flow rates (mL/min) | $Q_{feed}$ | 2.42 |
|---|---|---|
|  | $Q_{raf}$ | 2.71 |
|  | $Q_{ext}$ | 4.71 |
|  | $Q_{des}$ | 5.00 |
| Zone flow rates (mL/min) | $Q_1$ | 5.00 |
|  | $Q_2$ | 7.42 |
|  | $Q_3$ | 4.71 |
| Switching time (min) |  | 21.33 |
| Valine Purity (%) |  | 98.0 |
| Valine yield (%) |  | 98.1 |

**Example 6: SMB Experiment**

By using the date obtained above, valine was separated by the continuous separation device of valine according to the present invention, and the yield and purity thereof were measured as follows:

1) Experimental method

As shown in Fig. 4, a SMB experiment was carried out by using the optimized conditions derived from above Example 5. Amberchrom CG161C was used as the column.

To begin, the column was connected prior to the start of the experiment. At this time, the flow rate of desorbent (water) was maintained as 2 ml/min, and the operation of the other pump was halted. Each of the valve and column were connected, while at this time care was taken such that no air is entered inside the column. After completing all the connections of the columns, the flow rate of the desorbent was increased up to the target value. Since the start of the SMB experiment is for the time that the mixture solution to be separated is injected, the pump of the mixture to be separated was operated concurrent to the operation of Labview 8.0 software. The switching and switching time of the valve were simultaneously controlled via Labview 8.0.

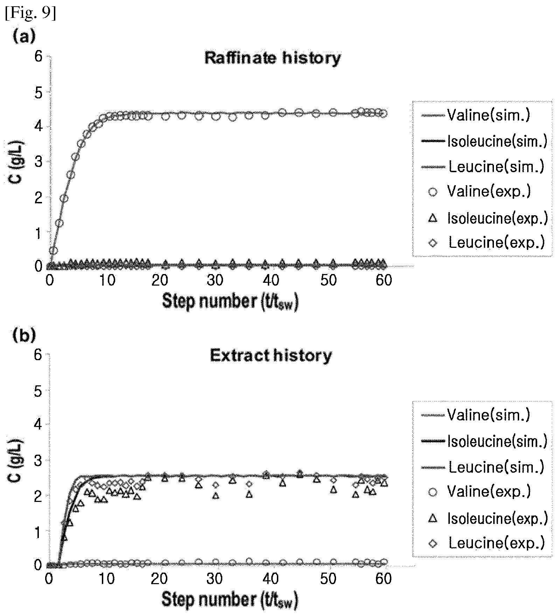

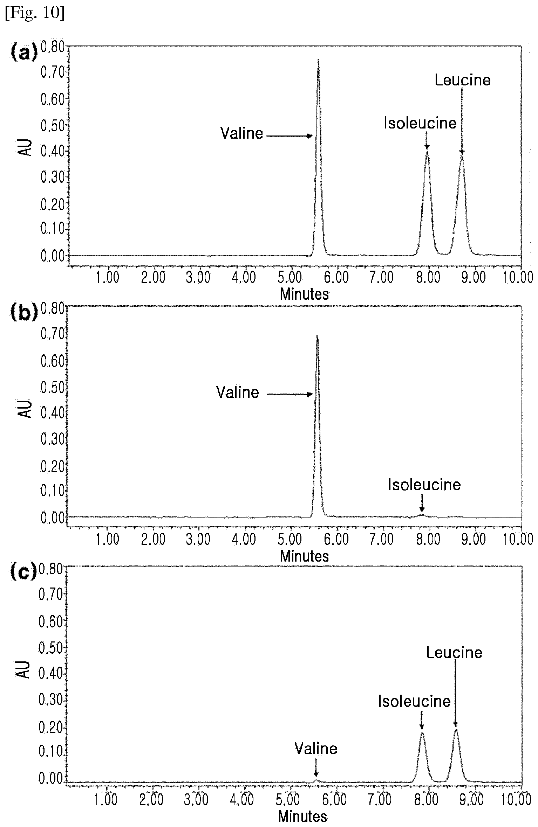

The SMB experiment was progressed until it sufficiently reached a cyclic steady state. It was ascertained that the steady state was achieved after the 25th step. Therefore, the SMB experiment progressed up to the 60th step, which is a value greatly in excess of that of the steady state. Throughout the SMB experiment, at every step, the concentration of the solution eluted from each outlet port was analyzed by using the HPLC device. Also, in order to secure the column profile, the relevant sample was taken at the last step. For the purpose of this, all driving pumps were stopped when the SMB experiment reached the 60.5 step. Additionally, the solution exiting from the column by opening the lower part of the column connected to the valve was collected, and the concentration of the solution was analyzed. HPLC analysis conditions were that: 10% aqueous methanol solution was used as the mobile phase, the injection volume was 20 $\mu\ell$, and the flow rate was 0.5 ml/min. Before the analysis, the self-calibration curve was secured by using the standard solution. The results are represented in Figure 9. In Figure 10, (a) is the analysis result of the raffinate concentration, and (b) is the analysis result of the extract concentration.

2) Experiment results

As shown in Figure 9a, it could be ascertained that most of the components in the raffinate solution were valine. Leucine and isoleucine, belonging to the impurities, were rarely present. This means that the purity of valine being maintained is very high. Also, as shown in Figure 9b from the analysis result of the extract concentration, most of the components were leucine and isoleucine, and the detected amount of valine was of a negligible quantity. This means that the loss of valine via the outlet port is minimized.

Also, the computer simulation for the relevant SMB process was carried out simultaneously with the progress of the SMB experiment, and the result was directly compared with the HPLC concentration analysis data of the raffinate and the extract. As shown in Figure 9, it can be seen that the simulation result and SMB experimental data correlated well.

In order to make a quantitative measure of the final purity and yield of valine separated by SMB experiment as mentioned above, the effluent solutions obtained during the last six steps were mixed in the same ratio and HPLC concentration analysis for the solution was then conducted. Based on the analyzed concentration data, the purity and yield of valine were calculated, and the results were represented in Table 3 as below.

Table 3

[Table 3]

|  | Experiment | Simulation |
|---|---|---|
| Yield (%) | 98.25 | 98.01 |
| Purity (%) | 97.50 | 98.03 |

From the results of Table 3 and Fig. 9, it could be ascertained that the SMB process using Amberchrom CG161C of the present invention is superior in assuring the continuous separation of valine and maintaining high purity and high yield throughout. As the experimental evidence data for this, a raw chromatogram of HPLC concentration analysis (HPLC raw data) was represented in Figure 10.

At first, in HPLC raw data (Fig. 10a), for the mixture solution to be separated all of three amino acid components represented large peaks. In HPLC raw data (Fig. 10b) for the Raffinate port effluent that belongs to the production port of valine, only the valine component clearly showed a large peak while the peak of isoleucine showed a very small peak, and further the peak of leucine was rarely found. In HPLC raw data (Fig. 10c) for the Extract port effluent which is established so as to obtain only data corresponding to the impurity, the peak of the valine component is negligible, while the two remaining amino acid components showed a large peak. Through this series of HPLC raw data, it could be ascertained again that the SMB experiment for the continuous separation of valine carried out as in the present invention was successfully conducted

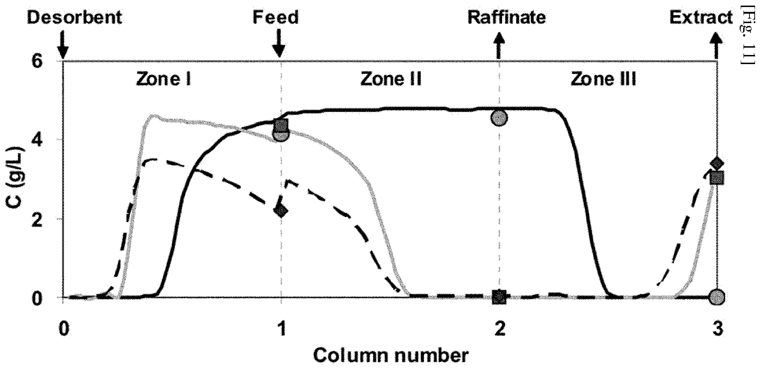

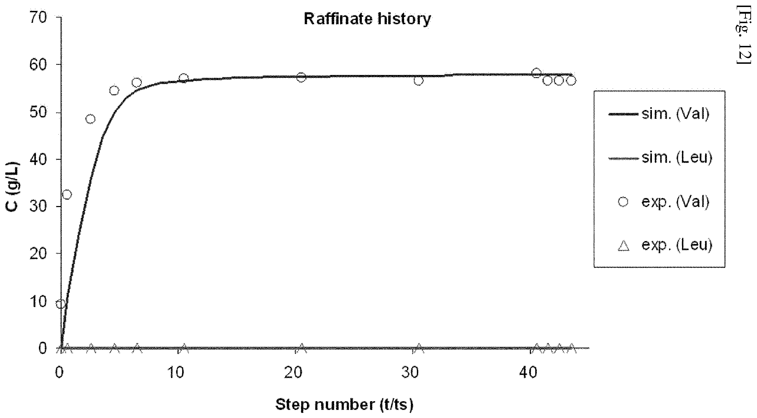

In addition to the concentration graphs of the raffinate and extract as mentioned above, the column profile data is also important SMB experimental data. For this reason, the samples required for securing the column profile data were taken during the halfway mark of the final switching period, that is, at step 60.5. After analyzing the sample concentration, the result was represented in Fig. 12. As can be seen from Fig. 11, it can be ascertained that the column profile data also correlates well with the simulation result. This discloses that the column profile proves the SMB experiment was conducted well. That is to say, it can be said that the facts as experimentally verified that solute waves of each of the amino acids in the SMB column were distributed so that they are fully advantageous to a high purity and yield.

Therefore, from all of the SMB experimental data (the concentration graph at the outlet port, column profile) mentioned above, it could be seen that the SMB process of the present invention using Amberchrom CG161C is sufficiently applicable to the continuous separation process of valine on an industrial scale.

The above mentioned SMB process was optimized based on Amberchrom CG161C adsorbent, and also verified experimentally. In addition to this adsorbent, SMB process based on Chromalite PCG-600 wherein the effect on the valine separation was verified was also conducted for optimization, and the experiment in this regard was also conducted. As a result, it was ascertained that Chromalite PCG-600 adsorbent was also sufficiently utilizable for the continuous separation of valine upon being applied to the SMB process.

**Example 7: Experiment in the actual fermentation mixture**

According to a method similar to that of the previous experiment, an experiment was carried out by using the actual fermentation mixture.

In order to measure the basic parameter (adsorption and mass transfer coefficient) of valine and leucine components in the actual fermentation mixture, a mixture frontal experiment was conducted wherein the solution of the fermentation mixture was injected into a single column filled with Chromalite PCG600C resin while the concentration of column effluent was measured over the time.

The basic parameters of valine and leucine were determined by using the concentration profile data obtained from the experiment and the inverse method, and the results are given in Table 4.

Table 4

[Table 4]

|  | Linear isotherm parameter | Intra-particle diffusivity ($cm^2$/min) | Molecular diffusivity ($cm^2$/min) |
|---|---|---|---|
| valine | 0.6065 | $1.20 \times 10^{-5}$ | $5.10 \times 10^{-4}$ |
| leucine | 3.925 | $1.75 \times 10^{-5}$ | $5.40 \times 10^{-4}$ |

On the basis of the basic parameters given in the above Table 4, the PCG600C-SMB process continuously separating valine and leucine from the actual fermentation mixture was optimally designed. This SMB process was also based on the new port disposition order of the present invention, and the column configuration adapted the three-zone structure as shown in Fig. 3.

The matter being focused upon in the optimization procedure of PCG600C-SMB process was the maximization of the productivity of valine while assuring a high yield of valine and a high removal efficiency of leucine. In order to achieve this optimization purpose, the productivity of valine was established as an objective function, and the yield of valine and the removal rates of leucine were established as the constraint to the conduct of the optimization procedure. The concrete optimization procedures are summarized below. $Q_{des}$ is the value which is controlled within the scope that the purity and yield of valine can be increased.

$$\text{Max } J = \text{Productivity } [Q_{feed}, Q_{raf}, t_{sw}]$$

Subject to Valine purity = 97%
Leucine removal efficiency = 90%
Fixed variables $Q_{des}$ = 6 mL/min
Lc = 21.7 cm, $d_C$ = 2.5 cm
Column configuration = 1 - 1 - 2
(wherein, $Q_{feed}$ and $Q_{des}$ refer to the flow rate of the mixture to be separated and the flow rate of the desorbent, respectively, and $t_{sw}$ refers to the switching time.)

In order to conduct the optimization of PCG600C-SMB process according to the above equation, the optimized computer program based on NSGA-II-JG algorithm was self-coded, and the computer program was connected to the Aspen simulator to obtain the optimization of the relevant process. The optimization results are given in Table 5.

Table 5

[Table 5]

| Inlet and outlet flow rates (mL/min) | $Q_{feed}$ | 3.27 |
|---|---|---|
| | $Q_{raf}$ | 3.93 |
| | $Q_{ext}$ | 5.34 |
| | $Q_{des}$ | 6.00 |
| Zone flow rates (mL/min) | $Q_1$ | 6.00 |
| | $Q_2$ | 9.27 |
| | $Q_3$ | 5.34 |
| Switching time (min) | | 17.85 |

In order to experimentally verify the optimization results of Table 5, PCG600C-SMB process device was self-assembled. The mimetic diagram of the assembled process device is given in Fig. 4.

The SMB experiment for separating valine wherein the actual fermentation mixture is the subject was conducted by using the optimization result of Table 2 and the process device of Fig. 4. The concentrations of valine and leucine in the actual fermentation mixture used in this experiment were 71.8 g/L and 0.742 g/L, respectively.

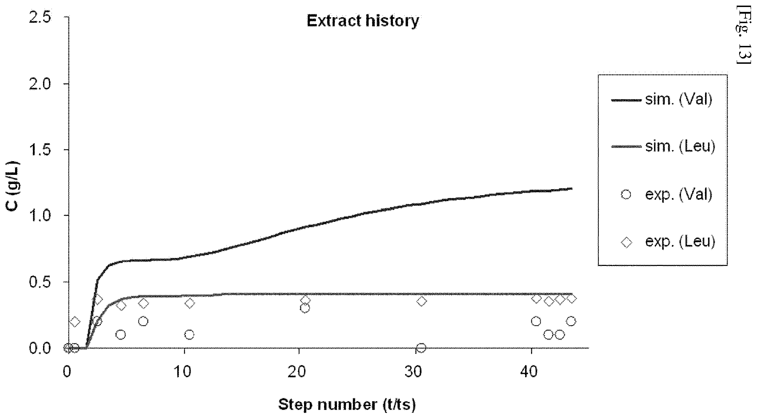

The results of the SMB experiment for separating valine, wherein the actual fermentation mixture was the subject are represented in Figs. 12-14. As shown in the result of the raffinate effluent history in Fig. 12, the high concentration recovery of valine was made in accordance with the level being expected by the simulation, and the effluent concentration level of leucine was low enough to the extent of being considered a negligible quantity. As shown in the result of the extract effluent history of Fig. 13, it could be ascertained that leucine is removed in accordance with the level expected by the simulation. Also, it could be ascertained that the loss of valine via an Extract port was minimized. These experimental results mean that a high yield of valine and a high removal rate of leucine can be sufficiently assured by the continuous separation of valine and leucine by the PCG600C-SMB process.

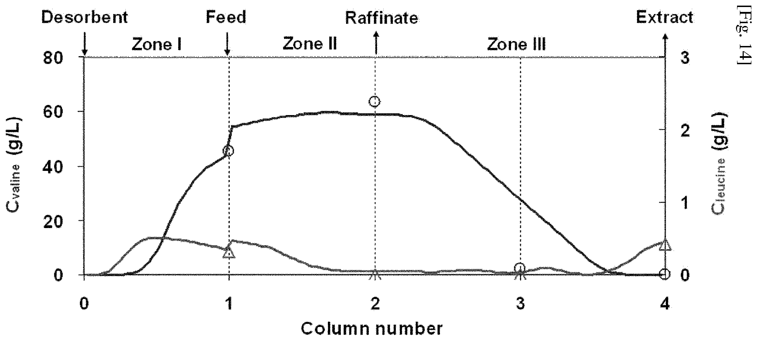

Further to the effluent history results of Figs. 12 and 13, the column profile result of Fig. 14 also comprehensively shows that the continuous separation of valine and leucine was successfully conducted. As shown in Fig. 14, it can be ascertained that the concentration distribution of valine and leucine in each column is made to be very advantageous in assuring a high yield recovery of valine and a high removal rate of leucine. Due to these results, 99.7 % of valine in the actual fermentation mixture could be recovered via the Raffinate port, and at the same time 98.0 % of leucine could be removed via the Extract port.

## Claims

1. A method for continuously separating valine using an apparatus for continuous separation of valine, wherein the apparatus comprises:

   a Desorbent port (D) configured to supply a desorbent to the apparatus;
   a Feed port (F) configured to supply a mixture including valine to the apparatus;
   a Raffinate port (R) configured to extract the valine separated from the mixture outside of the apparatus:

      an Extract port (E) configured to extract the other materials separated from the mixture outside of the apparatus;
      a first rotary valve (10),
      a second rotary valve (20) and
      a third rotary valve (30);
      and a first chromatography zone (40),
      a second chromatography zone (50) and
      a third chromatography zone (60),

   wherein upstream side of the first chromatography zone (40) and downstream side of the third chromatography zone (60) are fluidly connected by the first rotary valve (10),

wherein downstream side of the first chromatography zone (40) and the upstream side of the second chromatography zone (50) are fluidly connected by the second rotary valve (20),

wherein downstream side of the second chromatography zone (50) and the upstream side of the third chromatography zone (60) are fluidly connected by the third rotary valve (30),

wherein the method performs sequentially a first operation, a second operation and a third operation for continuous separation of valine,

wherein the method comprises:

in the first operation, fluidly connecting the Desorbent port (D) to the upstream side of the first chromatography zone (40) by the first rotary valve (10), fluidly connecting the Feed port (F) to the upstream side of the second chromatography zone (50) by the second rotary valve (20), fluidly connecting the Raffinate port (R) to the downstream side of the second chromatography zone (50) by the third rotary valve (30), and fluidly connecting the Extract port (E) to the downstream side of the third chromatography zone (60) by the first rotary valve (10);

in the second operation, fluidly connecting the Desorbent port (D) to the upstream side of the second chromatography zone (50) by the second rotary valve (20), fluidly connecting the Feed port (F) to the upstream side of the third chromatography zone (60) by the third rotary valve (30), fluidly connecting the Raffinate port (R) to the downstream side of the third chromatography zone (60) by the first rotary valve (10), and fluidly connecting the Extract port (E) to the downstream side of the first chromatography zone (40) by the second rotary valve (20); and

in the third operation, fluidly connecting the Desorbent port (D) to the upstream side of the third chromatography zone (60) by the third rotary valve (30), fluidly connecting the Feed port (F) to the upstream side of the first chromatography zone (40) by the first rotary valve (10), fluidly connecting the Raffinate port (R) to the downstream side of the first chromatography zone (40) by the second rotary valve (20), and fluidly connecting the Extract port (E) to the downstream side of the second chromatography zone (50) by the third rotary valve (30),

wherein the adsorbent used in said first, second and third chromatography zone is an insoluble polystyrene divinylbenzene polymer having a diameter of a particle of 10 - 300 $\mu$m,

wherein the mixture includes at least one of leucine and isoleucine as the other materials,

wherein the purity of the valine recovered is 90% to 99%.

2. The method for continuously separating valine according to claim 1,

wherein the first rotary valve comprises first, second and third connection ports (10a, 10b, 10c),

wherein the method comprises:

in the first operation, opening the first connection port (10a) so that the Desorbent port (D) is fluidly connected to the upstream side of the first chromatography zone (40) and the Extract port (E) is fluidly connected to the downstream side of the third chromatography zone (60), and closing the second and third connection ports (10b, 10c);

in the second operation, opening the second connection port (10b) so that the Raffinate port (R) is fluidly connected to the downstream side of the third chromatography zone (60), and closing the first and third connection ports (10a, 10c);

and in the third operation, opening the third connection port (10c) so that the Feed port (F) is fluidly connected to the upstream side of the first chromatography zone (40), and closing the first and second connection ports (10a, 10b).

3. The method for continuously separating valine according to claims 1 or 2,

wherein the second rotary valve comprises first, second and third connection ports (20a, 20b, 20C),

wherein the method comprises:

in the first operation, opening the first connection port (20a) so that the Feed port (F) is fluidly connected to the upstream side of the second chromatography zone (50), and closing the second and third connection ports (20b, 20c);

in the second operation, opening the second connection port (20b) so that the Desorbent port (D) is fluidly connected to the upstream side of the second chromatography zone (50) and the Extract port (E) is fluidly connected to the downstream side of the first chromatography zone (40) by the second rotary valve (20), and closing the first and third connection ports (20a, 20c); and
in the third operation, opening the third connection port (20c) so that the Raffinate port (R) is fluidly connected to the downstream side of the first chromatography zone (40), and closing the first and second connection ports (20a, 20b).

**4.** The method for continuously separating valine according to anyone of claims 1 - 3,
wherein the third rotary valve comprises first, second and third connection ports (30a, 30b, 30c),
wherein the method comprises:

in the first operation, opening the first connection port (30a) so that the Raffinate port (R) is fluidly connected to the downstream side of the second chromatography zone (50), and closing the second and third connection ports (30b, 30c);
in the second operation, opening the second connection port (30b) so that the Feed port (F) is fluidly connected to the upstream side of the third chromatography zone (60), and closing the first and third connection ports (30a, 30c); and

in the third operation, the third connection port (30c) so that the Desorbent port (D) is fluidly connected to the upstream side of the third chromatography zone (60) and the Extract port (E) is fluidly connected to the downstream side of the second chromatography zone (50), and closing the first and second connection ports (30a, 30b).

**Patentansprüche**

**1.** Verfahren zur kontinuierlichen Abscheidung von Valin unter Verwendung eines Geräts zur kontinuierlichen Abscheidung von Valin,
wobei das Gerät umfasst:

eine Desorptionsmittel-Öffnung (D) konfiguriert zum Zuführen eines Desorptionsmittels zu dem Gerät;
eine Beschickungs-Öffnung (F) konfiguriert zum Zuführen eines Valin einschließenden Gemisches zu dem Gerät;
eine Raffinat-Öffnung (R) konfiguriert zum Entnehmen des von dem Gemisch abgeschiedenen Valins außerhalb des Geräts;
eine Entnahme-Öffnung (E) konfiguriert zum Entnehmen der anderen von dem Gemisch abgeschiedenen Materialien außerhalb des Geräts;
ein erstes Drehventil (10),
ein zweites Drehventil (20) und
ein drittes Drehventil (30);
und eine erste Chromatografiezone (40),
eine zweite Chromatografiezone (50) und
eine dritte Chromatografiezone (60),
wobei die der ersten Chromatografiezone (40) vorgelagerte Seite und die der dritten Chromatografiezone (60) nachgelagerte Seite über das erste Drehventil (10) fluidisch verbunden sind,
wobei die der ersten Chromatografiezone (40) nachgelagerte Seite und die der zweiten Chromatografiezone (50) vorgelagerte Seite über das zweite Drehventil (20) fluidisch verbunden sind,
wobei die der zweiten Chromatografiezone (50) nachgelagerte Seite und die der dritten Chromatografiezone (60) vorgelagerte Seite über das dritte Drehventil (30) fluidisch verbunden sind,
wobei das Verfahren der Reihe nach einen ersten Vorgang, einen zweiten Vorgang und
einen dritten Vorgang zum kontinuierlichen Abscheiden von Valin durchführt,
wobei das Verfahren umfasst:

in dem ersten Vorgang, fluidisch Verbinden der Desorptionsmittel-Öffnung (D) mit der der ersten Chromatografiezone (40) vorgelagerten Seite über das erste Drehventil (10), fluidisch Verbinden der Beschickungs-Öffnung (F) mit der der zweiten Chromatografiezone (50) vorgelagerten Seite über das zweite Drehventil (20), fluidisch Verbinden der Raffinat-Öffnung (R) mit der der zweiten Chromatografiezone (50) nachgelagerten Seite über das dritte Drehventil (30), und fluidisch Verbinden der Entnahme-Öffnung (E) mit der der dritten Chromatografiezone (60) nachgelagerten Seite über das erste Drehventil (10);
in dem zweiten Vorgang, fluidisch Verbinden der Desorptionsmittel-Öffnung (D) mit der der zweiten Chromatografiezone (50) vorgelagerten Seite über das zweite Drehventil (20), fluidisch Verbinden der Beschickungs-Öffnung (F) mit der der dritten Chromatografiezone (60) vorgelagerten Seite über das dritte Drehventil (30), fluidisch Verbinden der Raffinat-Öffnung (R) mit der der dritten Chromatografiezone (60) nachgelagerten Seite über das erste Drehventil (10), und fluidisch Verbinden der Entnahme-Öffnung (E) mit der der ersten Chromatografiezone (40) nachgelagerten Seite über das zweite Drehventil (20); und

in dem dritten Vorgang, fluidisch Verbinden der Desorptionsmittel-Öffnung (D) mit der der dritten Chromatografiezone (60) vorgelagerten Seite über das dritte Drehventil (30), fluidisch Verbinden der Beschickungs-Öffnung (F) mit der der ersten Chromatografiezone (40) vorgelagerten Seite über das erste Drehventil (10), fluidisch Verbinden der Raffinat-Öffnung (R) mit der der ersten Chromatografiezone (40) nachgelagerten Seite über das zweite Drehventil (20), und fluidisch Verbinden der Entnahme-Öffnung (E) mit der der zweiten Chromatografiezone (50) nachgelagerten Seite über das dritte Drehventil (30),

wobei das in der ersten, zweiten und dritten Chromatografiezone verwendete Adsorptionsmittel ein unlösliches Polystyrendivinylbenzen-Polymer mit einem Teilchendurchmesser von 10 - 300 μm ist,

wobei das Gemisch mindestens eines von Leucin und Isoleucin als die anderen Materialien einschließt,

wobei die Reinheit des gewonnenen Valins 90% bis 99% beträgt.

2. Verfahren zur kontinuierlichen Abscheidung von Valin gemäß Anspruch 1, wobei das erste Drehventil erste, zweite und dritte Verbindungsöffnungen (10a, 10b, 10c) umfasst, wobei das Verfahren umfasst:

in dem ersten Vorgang, Öffnen der ersten Verbindungsöffnung (10a), so dass die Desorptionsmittel-Öffnung (D) fluidisch mit der der ersten Chromatografiezone (40) vorgelagerten Seite verbunden ist und die Entnahme-Öffnung (E) fluidisch mit der der dritten Chromatografiezone (60) nachgelagerten Seite verbunden ist, und Schließen der zweiten und dritten Verbindungsöffnungen (10b, 10c);

in dem zweiten Vorgang, Öffnen der zweiten Verbindungsöffnung (10b), so dass die Raffinat-Öffnung (R) fluidisch mit der der dritten Chromatografiezone (60) nachgelagerten Seite verbunden ist, und Schließen der ersten und dritten Verbindungsöffnungen (10a, 10c);

und in dem dritten Vorgang, Öffnen der dritten Verbindungsöffnung (10c), so dass die Beschickungs-Öffnung (F) fluidisch mit der der ersten Chromatografiezone (40) vorgelagerten Seite verbunden ist, und Schließen der ersten und zweiten Verbindungsöffnungen (10a, 10b).

3. Verfahren zur kontinuierlichen Abscheidung von Valin gemäß den Ansprüchen 1 oder 2, wobei das zweite Drehventil erste, zweite und dritte Verbindungsöffnungen (20a, 20b, 20c) umfasst, wobei das Verfahren umfasst:

in dem ersten Vorgang, Öffnen der ersten Verbindungsöffnung (20a), so dass die Beschickungs-Öffnung (F) fluidisch mit der der zweiten Chromatografiezone (50) vorgelagerten Seite verbunden ist, und Schließen der zweiten und dritten Verbindungsöffnungen (20b, 20c);

in dem zweiten Vorgang, Öffnen der zweiten Verbindungsöffnung (20b), so dass über das zweite Drehventil (20) die Desorptionsmittel-Öffnung (D) fluidisch mit der der zweiten Chromatografiezone (50) vorgelagerten Seite verbunden ist und die Entnahme-Öffnung (E) fluidisch mit der der ersten Chromatografiezone (40) nachgelagerten Seite verbunden ist, und Schließen der ersten und dritten Verbindungsöffnungen (20a, 20c); und

in dem dritten Vorgang, Öffnen der dritten Verbindungsöffnung (20c), so dass die Raffinat-Öffnung (R) fluidisch mit der der ersten Chromatografiezone (40) nachgelagerten Seite verbunden ist, und Schließen der ersten und zweiten Verbindungsöffnungen (20a, 20b).

4. Verfahren zur kontinuierlichen Abscheidung von Valin gemäß einem der Ansprüche 1 - 3, wobei das dritte Drehventil erste, zweite und dritte Verbindungsöffnungen (30a, 30b, 30c) umfasst, wobei das Verfahren umfasst:

in dem ersten Vorgang, Öffnen der ersten Verbindungsöffnung (30a), so dass die Raffinat-Öffnung (R) fluidisch mit der der zweiten Chromatografiezone (50) nachgelagerten Seite verbunden ist, und Schließen der zweiten und dritten Verbindungsöffnungen (30b, 30c);

in dem zweiten Vorgang, Öffnen der zweiten Verbindungsöffnung (30b), so dass die Beschickungs-Öffnung (F) fluidisch mit der der dritten Chromatografiezone (60) vorgelagerten Seite verbunden ist, und Schließen der ersten und dritten Verbindungsöffnungen (30a, 30c); und

in dem dritten Vorgang, Öffnen der dritten Verbindungsöffnung (30c), so dass die Desorptionsmittel-Öffnung (D) fluidisch mit der der dritten Chromatografiezone (60) vorgelagerten Seite verbunden ist und die Entnahme-Öffnung (E) fluidisch mit der der zweiten Chromatografiezone (50) nachgelagerten Seite verbunden ist, und Schließen der ersten und zweiten Verbindungsöffnungen (30a, 30b).

## Revendications

1. Procédé de séparation continue de valine au moyen d'un appareil de séparation continue de valine, dans lequel l'appareil comprend :

   un orifice de désorbant (D) configuré pour fournir un désorbant à l'appareil ;
   un orifice d'alimentation (F) configuré pour fournir un mélange incluant de la valine à l'appareil ;
   un orifice de raffinat (R) configuré pour extraire la valine séparée à partir du mélange hors de l'appareil ;
   un orifice d'extrait (E) configuré pour extraire les autres matières séparées à partir du mélange hors de l'appareil ;
   une première vanne rotative (10),
   une deuxième vanne rotative (20), et
   une troisième vanne rotative (30) ;
   et une première zone de chromatographie (40),
   une deuxième zone de chromatographie (50) et
   une troisième zone de chromatographie (60),
   dans lequel un côté amont de la première zone de chromatographie (40) et un côté aval de la troisième zone de chromatographie (60) sont raccordés de manière fluidique par la première vanne rotative (10),
   dans lequel un côté aval de la première zone de chromatographie (40) et un côté amont de la deuxième zone de chromatographie (50) sont raccordés de manière fluidique par la deuxième vanne rotative (20),
   dans lequel un côté aval de la deuxième zone de chromatographie (50) et un côté amont de la troisième zone de chromatographie (60) sont raccordés de manière fluidique par la troisième vanne rotative (30),
   dans lequel le procédé effectue de manière séquentielle une première opération, une deuxième opération et une troisième opération pour une séparation continue de valine,
   dans lequel le procédé comprend :

   dans la première opération, le raccordement de manière fluidique de l'orifice de désorbant (D) au côté amont de la première zone de chromatographie (40) par la première vanne rotative (10), le raccordement de manière fluidique de l'orifice d'alimentation (F) au côté amont de la deuxième zone de chromatographie (50) par la deuxième vanne rotative (20), le raccordement de manière fluidique de l'orifice de raffinat (R) au côté aval de la deuxième zone de chromatographie (50) par la troisième vanne rotative (30), et le raccordement de manière fluidique de l'orifice d'extrait (E) au côté aval de la troisième zone de chromatographie (60) par la première vanne rotative (10) ;
   dans la deuxième opération, le raccordement de manière fluidique de l'orifice de désorbant (D) au côté amont de la deuxième zone de chromatographie (50) par la deuxième vanne rotative (20), le raccordement de manière fluidique de l'orifice d'alimentation (F) au côté amont de la troisième zone de chromatographie (60) par la troisième vanne rotative (30), le raccordement de manière fluidique de l'orifice de raffinat (R) au côté aval de la troisième zone de chromatographie (60) par la première vanne rotative (10), et le raccordement de manière fluidique de l'orifice d'extrait (E) au côté aval de la première zone de chromatographie (40) par la deuxième vanne rotative (20) ; et
   dans la troisième opération, le raccordement de manière fluidique de l'orifice de désorbant (D) au côté amont de la troisième zone de chromatographie (60) par la troisième vanne rotative (30), le raccordement de manière fluidique de l'orifice d'alimentation (F) au côté amont de la première zone de chromatographie (40) par la première vanne rotative (10), le raccordement de manière fluidique de l'orifice de raffinat (R) au côté aval de la première zone de chromatographie (40) par la deuxième vanne rotative (20), et le raccordement de manière fluidique de l'orifice d'extrait (E) au côté aval de la deuxième zone de chromatographie (50) par la troisième vanne rotative (30),
   dans lequel l'adsorbant utilisé dans lesdites première, deuxième et troisième zones de chromatographie est un polymère de polystyrène divinylbenzène insoluble présentant un diamètre de particule de 10 à 300 $\mu$m,
   dans lequel le mélange inclut au moins l'une parmi la leucine et l'isoleucine en tant qu'autres matières,
   dans lequel la pureté de la valine récupérée est de 90 % à 99 %.

2. Procédé de séparation continue de valine selon la revendication 1, dans lequel la première vanne rotative comprend des premier, deuxième et troisième orifices de raccordement (10a, 10b, 10c),

dans lequel le procédé comprend :

dans la première opération, l'ouverture du premier orifice de raccordement (10a) de sorte que l'orifice de désorbant (D) soit raccordé de manière fluidique au côté amont de la première zone de chromatographie (40) et que l'orifice d'extrait (E) soit raccordé de manière fluidique au côté aval de la troisième zone de chromatographie (60), et la fermeture des deuxième et troisième orifices de raccordement (10b, 10c) ;
dans la deuxième opération, l'ouverture du deuxième orifice de raccordement (10b) de sorte que l'orifice de raffinat (R) soit raccordé de manière fluidique au côté aval de la troisième zone de chromatographie (60), et la fermeture des premier et troisième orifices de raccordement (10a, 10c) ;
et dans la troisième opération, l'ouverture du troisième orifice de raccordement (10c) de sorte que l'orifice d'alimentation (F) soit raccordé de manière fluidique au côté amont de la première zone de chromatographie (40), et la fermeture des premier et deuxième orifices de raccordement (10a, 10b).

3. Procédé de séparation continue de valine selon les revendications 1 ou 2,
dans lequel la deuxième vanne rotative comprend des premier, deuxième et troisième orifices de raccordement (20a, 20b, 20c),
dans lequel le procédé comprend :

dans la première opération, l'ouverture du premier orifice de raccordement (20a) de sorte que l'orifice d'alimentation (F) soit raccordé de manière fluidique au côté amont de la deuxième zone de chromatographie (50), et la fermeture des deuxième et troisième orifices de raccordement (20b, 20c) ;
dans la deuxième opération, l'ouverture du deuxième orifice de raccordement (20b) de sorte que l'orifice de désorbant (D) soit raccordé de manière fluidique au côté amont de la deuxième zone de chromatographie (50) et que l'orifice d'extrait (E) soit raccordé de manière fluidique au côté aval de la première zone de chromatographie (40) par la deuxième vanne rotative (20), et la fermeture des premier et troisième orifices de raccordement (20a, 20c) ; et
dans la troisième opération, l'ouverture du troisième orifice de raccordement (20c) de sorte que l'orifice de raffinat (R) soit raccordé de manière fluidique au côté aval de la première zone de chromatographie (40), et la fermeture des premier et deuxième orifices de raccordement (20a, 20b).

4. Procédé de séparation continue de valine selon l'une quelconque des revendications 1-3,
dans lequel la troisième vanne rotative comprend des premier, deuxième et troisième orifices de raccordement (30a, 30b, 30c),
dans lequel le procédé comprend :

dans la première opération, l'ouverture du premier orifice de raccordement (30a) de sorte que l'orifice de raffinat (R) soit raccordé de manière fluidique au côté aval de la deuxième zone de chromatographie (50), et la fermeture des deuxième et troisième orifices de raccordement (30b, 30c) ;
dans la deuxième opération, l'ouverture du deuxième orifice de raccordement (30b) de sorte que l'orifice d'alimentation (F) soit raccordé de manière fluidique au côté amont de la troisième zone de chromatographie (60), et la fermeture des premier et troisième orifices de raccordement (30a, 30c) ; et
dans la troisième opération, l'ouverture du troisième orifice de raccordement (30c) de sorte que l'orifice de désorbant (D) soit raccordé de manière fluidique au côté amont de la troisième zone de chromatographie (60) et que l'orifice d'extrait (E) soit raccordé de manière fluidique au côté aval de la deuxième zone de chromatographie (50), et la fermeture des premier et deuxième orifices de raccordement (30a, 30b).

[Fig. 1]

Desorbent     Extract     Feed     Raffinate

Zone I     Zone II     Zone III     Zone IV

[Fig. 2]

Desorbent     Extract     Feed     Raffinate

Zone I     Zone II     Zone III

[Fig. 3]

Desorbent     Feed     Raffinate     Extract

Zone I     Zone II     Zone III

[Fig. 4a]

[Fig. 4b]

[Fig. 4c]

EP 2 812 309 B1

[Fig. 5]

21

[Fig. 6]

[Fig. 7]

(a) Valine

(b) Leucine

(c) Isoleucine

[Fig. 8]

[Fig. 9]

**(a)**

Raffinate history

**(b)**

Extract history

[Fig. 10]

[Fig. 11]

[Fig. 12]

EP 2 812 309 B1

[Fig. 13]

Extract history

[Fig. 14]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 62255453 A
- JP 8333312 A
- JP 10237030 A
- US 6072083 A
- US 4263450 A
- CN 101948399 A

### Non-patent literature cited in the description

- **KANG, S.H. et al.** *Process Biochem.,* 2010, vol. 45, 1468-1476
- **LEE, K.B. et al.** *AIChE J.,* 2008, vol. 54, 2852-2871
- **NAM, H.G. et al.** *Process Biochem.,* 2011, vol. 46, 2044-2053
- **CHUNG, S. F. et al.** *AIChE J.,* 1968, vol. 14, 857-866
- **WILSON, E. J. et al.** *Ind. Eng. Chem. Fundam.,* 1966, vol. 5, 9-14
- **WILKE, C.R. et al.** *AIChE J.,* 1955, vol. 1, 264-270